# EUROPÄISCHE PATENTSCHRIFT

(11) **EP 3 644 877 B1**
(45) Veröffentlichungstag und Bekanntmachung des Hinweises auf die Patenterteilung: **19.07.2023**
(21) Anmeldenummer: 18734476.7
(22) Anmeldetag: 15.06.2018
(51) Int. Cl.: A61B 17/70, A61B 90/00

(54) **VERLÄNGERUNGSVORRICHTUNG FÜR EINEN KNOCHENANKER**
EXTENSION DEVICE FOR A BONE ANCHOR
DISPOSITIF DE PROLONGEMENT POUR UN ANCRAGE OSSEUX

(30) Priorität: 27.06.2017 DE 102017114273
(43) Veröffentlichungstag der Anmeldung: 06.05.2020
(73) Patentinhaber: Silony Medical International AG, 8500 Frauenfeld (CH)
(72) Erfinder: HEUER, Frank, 70794 Filderstadt (DE); SANDERS, Marc, 6241 GC Bunde (NL)
(74) Vertreter: DREISS Patentanwälte PartG mbB
(86) Internationale Anmeldenummer: PCT/EP2018/065987
(87) Internationale Veröffentlichungsnummer: WO 2019/001990

(56) Entgegenhaltungen:
- US-A1- 2009 228 054
- US-A1- 2010 137 875
- US-A1- 2011 263 945
- US-A1- 2015 066 089
- US-B1- 8 439 924

## Beschreibung

Die Erfindung betrifft eine Verlängerungsvorrichtung für einen Knochenanker, insbesondere für eine Knochenschraube, insbesondere für eine Pedikelschraube, insbesondere in der minimalinvasiven Wirbelsäulenchirurgie, mit einer axialen Längsrichtung, einer hierzu konzentrischen Umfangsrichtung und einer radialen Richtung, wobei die Verlängerungsvorrichtung in der axialen Längsrichtung erstreckt ist und mittels eines radialen Vorsprungs einen hintergreifbaren Bereich am Kopf des Knochenankers in der Längsrichtung und zudem in der radialen Richtung hintergreift und in entgegengesetzter Richtung gegen den Kopf des Knochenankers abstützbar ist, so dass die Verlängerungsvorrichtung hierdurch am Kopf des Knochenankers lösbar, jedoch in der Längsrichtung starr und zudem drehfest fixierbar ist.

Verlängerungsvorrichtungen der vorstehend genannten Art finden bevorzugt bei minimalinvasiven Eingriffen, insbesondere zur Implantation von Knochenankern Verwendung. Sie sollen während des chirurgischen Eingriffs gewissermaßen einen Arbeitskanal zu dem Knochenanker zugänglich halten, durch den der Chirurg mittels weiterer Instrumente den Knochenanker befestigen und gegebenenfalls weitere Implantatteile zuführen und befestigen kann und repositionierende Maßnahmen ausführen kann.

US 2011/263945 A1 und US 2010/137875 A1 und US 8,439,924 B1 zeigen jeweils Verlängerungsvorrichtungen mit zwei Schalenteilen, die aber einstückig miteinander verbunden sind. Die Schalenteile sind überwiegend im wesentlichen starr ausgebildet, sie weisen jedoch einen deformierbaren Bereich auf, der jeweils ein in der axialen Längsrichtung erstrecktes und quer hierzu auslenkbares Federelement bildet. Das jeweilige Federelement trägt einen radialen Vorsprung zum Hintergreifen eines hintergreifbaren Bereichs am Kopf des Knochenankers in der axialen Richtung und ferner einen nach radial innen vorstehenden Abstützbereich, mittels dessen das Federelement und damit der Schalenteil in axialer Richtung gegen den Kopf des Knochenankers abstützbar ist. Hierbei wird eine Fixierung der Verlängerungsvorrichtung und deren Schalenteile durch axialen Hintergriff mit dem Kopf des Knochenankers realisiert. Zum Lösen der Verlängerungsvorrichtung wird das jeweilige Federelement nach radial außen ausgelenkt, so dass der Vorsprung aus dem hintergreifbaren Bereich am Kopf des Knochenankers frei kommt und damit die Verlängerungsvorrichtung in der Längsrichtung in proximaler Richtung vom Kopf des Knochenankers abgezogen werden kann.

Eine Verlängerungsvorrichtung mit zwei voneinander separaten Schalenteilen ist beispielsweise bekannt aus WO 2007/021588 A1. Diese Schalenteile müssen mittels eines weiteren beide Teile verbindenden Montageteils am Kopf des Knochenankers befestigt werden. Ein radialer Hintergriff der Schalenteile am Kopf des Knochenankers scheint nicht vorgesehen zu sein.

Der vorliegenden Erfindung liegt die Aufgabe zugrunde, eine Verlängerungsvorrichtung für einen Knochenanker zu schaffen, die auf einfache Weise vor oder nach der Implantation des Knochenankers am Kopf des Knochenankers lösbar festlegbar ist, wobei ein unbeabsichtigtes Ablösen der Verlängerungsvorrichtung sicher vermieden werden können soll.

Diese Aufgabe wird durch eine Verlängerungsvorrichtung mit den Merkmalen des Anspruchs 1 gelöst.

Obschon vorzugsweise zwei Schalenteile zum Einsatz gelangen, damit diese beiden Schalenteile einen Arbeitskanal bilden können, ist es grundsätzlich denkbar, in bestimmten Situationen mit nur einem Schalenteil zu arbeiten. Werden zwei Schalenteile eingesetzt, so erweist es sich als vorteilhaft, wenn sie beide entsprechend der Erfindung und vorzugsweise identisch bzw. einander entsprechend ausgebildet sind.

Die erfindungsgemäße Verlängerungsvorrichtung bzw. deren jeweiliges Schalenteil ist am Kopf der Knochenschraube fixierbar, ohne dass zum unmittelbaren Halten eine Kraft erforderlich ist. Die Fixierung oder Halteverbindung ist vielmehr über einen Formschluss bezüglich aller Freiheitsgrade realisiert. Dadurch, dass der erste deformierbare Bereich bzw. das von ihm gebildete Federelement quer zur Längsrichtung auslenkbar ist, jedoch einen nach radial innen vorstehenden Abstützbereich aufweist, mittels dessen eine Abstützung in axialer Längsrichtung gegen den Kopf des Knochenankers in der Halteverbindung ausgeübt ist, ist das Schalenteil in axialer Richtung unverlierbar am Kopf des Knochenankers fixiert, da durch die axiale Abstützung verhindert ist, dass sich der radiale Vorsprung des Schalenteils aus dem hintergreifbaren Bereich am Kopf des Knochenankers in distaler Richtung löst. Durch den Abstützbereich kann zugleich eine formschlüssige Halterung in Umfangsrichtung erzielt werden. Es ist aber auch denkbar, dass dies alternativ oder zusätzlich in anderer Weise realisiert ist, also z.B. durch ein weiteres Federelement oder durch seitliche nach innen eingezogene Längsrandbereiche des Schalenteils, welche an Längsflanken der Schenkel des Kopfs des Knochenankers anliegen, oder über den radialen Vorsprung des Schalenteils selbst, der in den entsprechend mit Umfangsanschlägen versehenen hintergreifbaren Bereich am Kopf des Knochenankers eingreift.

Zum Fügen, d.h. zum Herstellen der lösbaren Halte- oder Fixierverbindung des jeweiligen Schalenteils mit dem Kopf des Knochenankers, wird nach einer Ausführungsvariante das Schalenteil am Außenumfang des Knochenankers positioniert, derart, dass der radiale Vorsprung des Schalenteils sich distal zu dem hintergreifbaren Bereich am Kopf des Knochenankers befindet. Dann wird das Schalenteil relativ zu dem Knochenanker in proximaler Richtung bewegt, bis der radiale Vorsprung in den hintergreifbaren Bereich am Kopf des Knochenankers eingreift oder einrastet und dabei auch die radiale Richtung hintergreift. In dieser Position kann das Federelement nach radial innen federnd einschwenken und damit der Abstützbereich des Federelements in eine axiale Abstützung am Kopf des Knochenankers gelangen. Somit ist das Schalenteil verliersicher am Kopf des Knochenankers gehalten. Zum Lösen dieser Halteverbindung muss das Federelement nach außen ausgelenkt werden, was den Einsatz eines hierfür speziell ausgebildeten Instruments vorzugsweise erfordert, um ein unbeabsichtigtes Lösen sicher zu verhindern.

Nach einer Ausführungsform der Erfindung erweist es sich als vorteilhaft, wenn das Federelement einstückig mit dem Schalenteil ausgebildet ist und an seinem proximalen Ende in eine Wandung des Schalenteils übergeht. Auf diese Weise kann das Federelement und das Schalenteil aus demselben Werkstoff gefertigt werden, eine anschließende Montage des Federelements ist nicht erforderlich.

Weiter erweist es sich als vorteilhaft, wenn das Federelement durch einen materialfreien Schlitz in der Wandung des Schadenteils begrenzt ist und an seinem proximalen Ende einstückig in die Wandung des Schalenteils übergeht.

Weiter erweist es sich als vorteilhaft, dass der materialfreie Schlitz proximal gekrümmt ausläuft, insbesondere mit einem Krümmungsradius von 0,5 - 5 mm. Hierdurch kann die Spannung beim Auslenken des Federelements gleichmäßiger in einen größeren Bereich in der Wandung des Schalenteils verteilt werden und eine frühzeitige Ermüdung verhindert werden. Alternativ oder zusätzlich hierzu erweist es sich als vorteilhaft, wenn der materialfreie Schlitz an seinem Ende in eine gegenüber der Schlitzbreite größere Öffnung ausmündet. Auch hierdurch können durch die Auslenkung des Federelements eingeleitete Kräfte gleichmäßiger verteilt werden.

Bei der Ausbildung des Federelements kann es sich als vorteilhaft erweisen, dass Federelement in einem Bereich seines Federarms proximal zu dem nach radial innen vorstehenden Abstützbereich eine in der axialen Längsrichtung in proximaler Richtung zunehmende Wanddicke aufweist. Insbesondere zusätzlich zu der vorgenannten Maßnahme kann es sich als vorteilhaft erweisen, dass das Federelement in einem Bereich seines Federarms proximal zu dem nach radial innen vorstehenden Abstützbereich eine in der axialen Längsrichtung in proximaler Richtung zunehmende Breite in der Umfangsrichtung aufweist. Weiter kann es sich als vorteilhaft erweisen, wenn das Federelement dadurch einstückig mit dem Schalenteil ausgebildet ist, dass es aus einer zuvor durchgehenden Wandung des Schalenteils durch einen Schnitt, Fräsen oder sonstige Materialabnahme freigeformt wurde. Alternativ hierzu sind additive Fertigungsverfahren, insbesondere 3D-Druck, denkbar, im Zuge derer das Schalenteil zusammen mit dem ersten deformierbaren Bereich bzw. mit dem Federelement hergestellt werden kann. Des Weiteren ist eine Kombination dieser Fertigungsverfahren denkbar. Als Werkstoffe sind metallische Legierungen sowie röntgendurchlässige Materialien denkbar.

Bei einer ganz besonders bevorzugten Ausführungsform der Erfindung wird vorgeschlagen, dass das Federelement in der Längsrichtung distal bis über den radialen Vorsprung zum Hintergreifen des hintergreifbaren Bereichs am Kopf des Knochenankers erstreckt ist und dort einen nach radial innen vorstehenden Eingriffsbereich aufweist, der in eine Eingriffsvertiefung am Kopf des Knochenankers einrastbar ist und eine zusätzliche formschlüssige Verbindung zwischen dem Schalenteil und dem Kopf des Knochenankers herstellen kann. Nach diesem Vorschlag bildet das Federelement nicht nur den axialen Abstützbereich, welcher proximal zu dem radialen Vorsprung vorgesehen ist, sondern es erstreckt sich zusätzlich weiter in distaler Richtung und weist dort den vorgenannten Eingriffsbereich auf, der einen zusätzlichen formschlüssigen Eingriff des Schalenteils mit dem Kopf des Knochenankers bewirkt. Diese zusätzliche formschlüssige Verbindung wird beim Auslenken des Federelements nach außen wieder gelöst, genauso wie die axiale Abstützung.

In weiterer Ausbildung dieser bevorzugten Ausführungsform erweist es sich als vorteilhaft, dass der radiale Vorsprung zum Hintergreifen des hintergreifbaren Bereichs am Kopf des Knochenankers in der Umfangsrichtung erstreckt ist, und dass dieser radiale Vorsprung und eine Wandung des Schalenteils im Bereich des Federelements in Umfangsrichtung unterbrochen oder ausgespart ist, damit das Federelement sich in der axialen Längsrichtung dort hindurch erstrecken kann. Es ist nämlich vorteilhaft, dass das Federelement aufgrund seiner distalen Erstreckung bis über den radialen Vorsprung diesen radialen Vorsprung durchbricht, damit das Federelement nicht radial außerhalb des Schalenteils nach außen aufträgt, was grundsätzlich auch denkbar wäre.

Weiter erweist es sich in diesem Zusammenhang als vorteilhaft, wenn das Federelement im Bereich des radialen Vorsprungs zum Hintergreifen des hintergreifbaren Bereichs am Kopf des Knochenankers in Umfangsrichtung verjüngt oder eingeschnürt ausgebildet ist. Solchenfalls wird die Unterbrechung des radialen Vorsprungs des Schalenteils in Umfangsrichtung geringer gehalten.

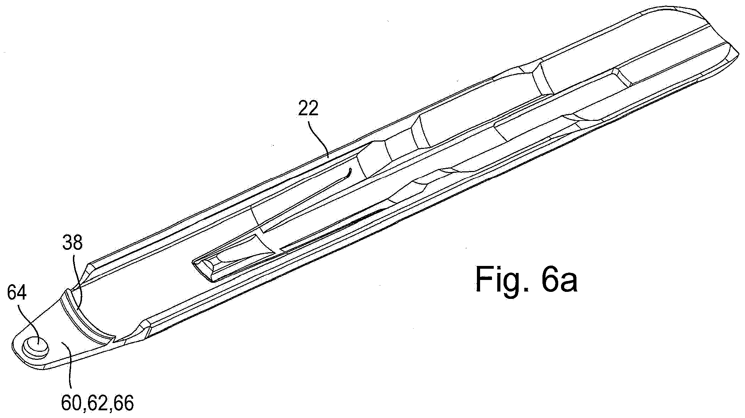

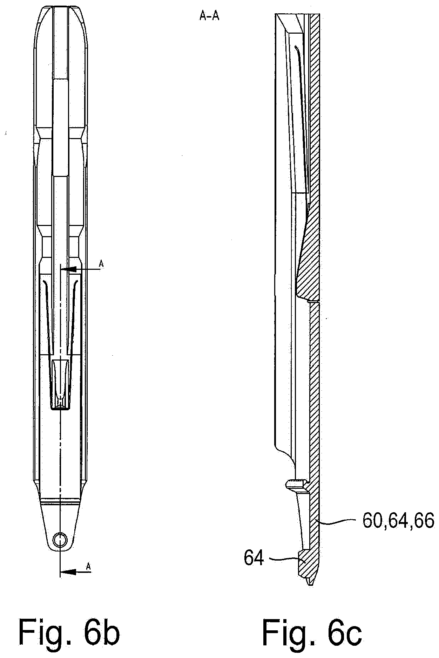

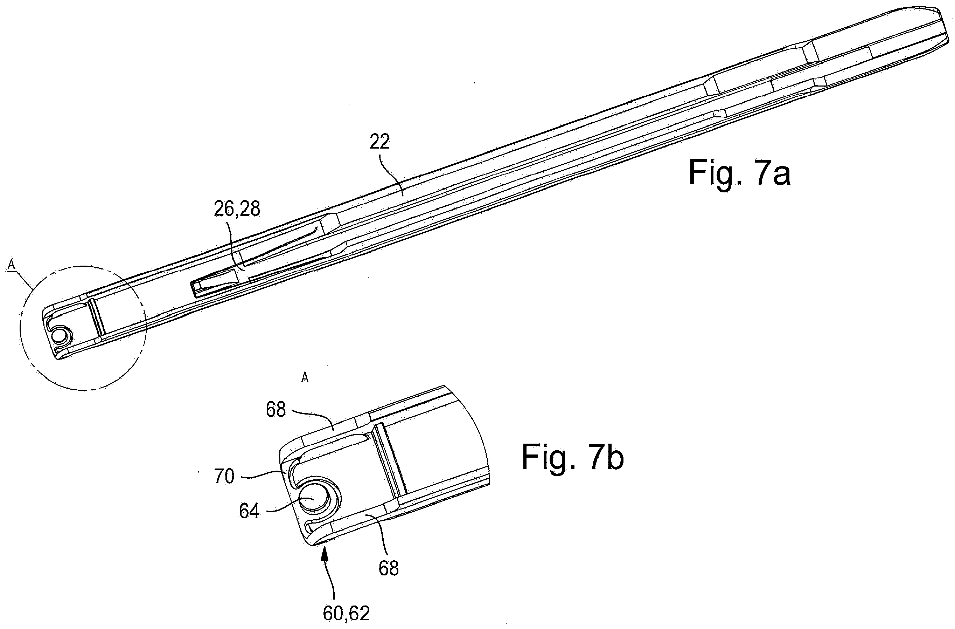

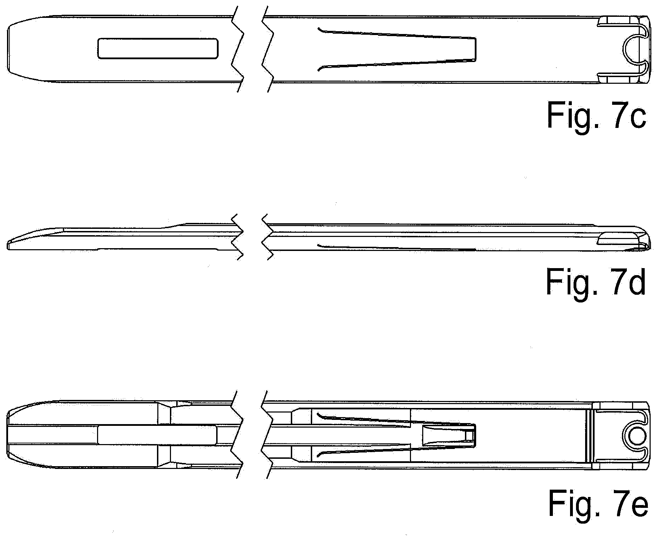

Nach einer weiteren Ausführungsform der Erfindung wird vorgeschlagen, dass das Schalenteil distal zu dem radialen Vorsprung zum Hintergreifen des hintergreifbaren Bereichs am Kopf des Knochenankers einen zweiten deformierbaren Bereich aufweist, der ein zweites Federelement bildet, wobei das zweite Federelement einen zweiten nach radial innen vorstehenden Eingriffsbereich aufweist, der in eine Eingriffsvertiefung am Kopf des Knochenankers einrastbar ist und eine zusätzliche formschlüssige Verbindung zwischen dem Schalenteil und dem Kopf des Knochenankers herstellen kann (Figuren 6, 7, 8). Bei dieser Ausführungsform kann das erste Federelement (im Unterschied zu der vorstehend erläuterten Ausführungsform) klar proximal zu dem radialen Vorsprung ausgebildet werden und insbesondere gegenüber der vorstehend beschriebenen Ausführungsform auch in proximaler Richtung von dem Vorsprung beabstandet angeordnet werden (vgl. Figuren 6, 7). Der zweite deformierbare Bereich und das von ihm gebildete zweite Federelement kann dann losgelöst von dem ersten Federelement distal zu dem radialen Vorsprung des Schalenteils ausgebildet werden.

In Weiterbildung dieser Ausführungsform kann es sich als vorteilhaft erweisen wenn der zweite deformierbare Bereich des Schalenteils von einem sich verjüngenden distalen Endabschnitt des Schalenteils gebildet ist. Alternativ oder zusätzlich hierzu kann es sich als vorteilhaft erweisen, wenn der zweite deformierbare Bereich, insbesondere der distale Endabschnitt des Schalenteils, Schwächungen, insbesondere in Form von Nuten oder Schlitzen, oder eine geringere Wandstärke als im wesentlichen starre Bereiche des Schalenteils aufweist.

Nach einer anderen Ausführungsform dieser Erfindungsvariante kann es sich als vorteilhaft erweisen, dass das zweite Federelement U-förmig ausgebildet ist und zwei Schenkel und einen diese verbindenden Quersteg aufweist, und dass der Eingriffsbereich an dem Quersteg ausgebildet ist und dass das Federelement mit Enden der Schenkel einstückig an das Schalenteil angebunden ist (Figuren 7).

Hierbei kann es sich als vorteilhaft erweisen, wenn die zwei Schenkel im wesentlichen in der Längsrichtung erstreckt sind und die Erstreckung des Schalenteils in der Längsrichtung fortsetzen.

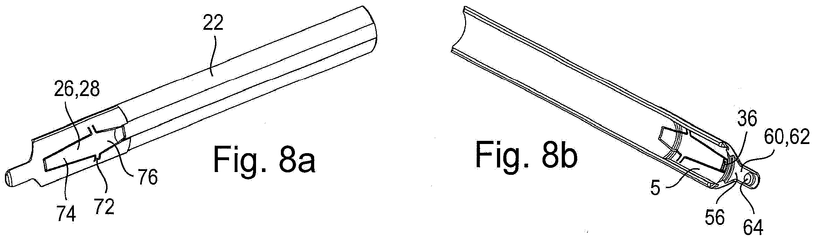

Nach einer weiteren Ausführungsform der erfindungsgemäßen Verlängerungsvorrichtung (Figuren 8) ist der erste deformierbare Bereich und das von ihm gebildete Federelement nach Art einer Wippe ausgebildet und weist einen bezüglich einer Schwenkachse distalen Wippenarm und einen bezüglich der Schwenkachse proximalen Wippenarm auf.

Bei dieser Ausführungsform kann es sich als vorteilhaft erweisen, wenn das Federelement in eine Freigabestellung bringbar ist, indem der proximale Wippenarm nach radial innen ausgelenkt wird, so dass hierdurch der distale Wippenarm nach radial außen ausgelenkt wird und von dem Kopf des Knochenankers frei kommt.

Bei den vorstehend beschriebenen erfindungsgemäßen Ausführungsformen ist der erste und gegebenenfalls der zweite deformierbare Bereich und das jeweilige Federelement vorzugsweise so ausgebildet und angeordnet, dass das Federelement durch die Federvorspannung in seine betreffende Halteverbindung am Kopf des Knochenankers einrastet. Dies kann durch Instrumente unterstützt werden. Zum Lösen der Halteverbindung muss also eine Auslenkbewegung auf das jeweilige Federelement ausgeübt werden. Dies erfolgt vorteilhafterweise und vorzugsweise zumindest bei dem ersten Federelement, indem ein Löseinstrument an das Federelement herangeführt wird. Hierfür erweist es sich als vorteilhaft, wenn das Schalenteil eine in der Längsrichtung erstreckte kanalbildende Zugangsöffnung aufweist, durch welche mittels des Löseinstruments Zugang zu dem Federelement genommen werden kann und das Federelement zum Lösen der Verlängerungsvorrichtung (in radialer Richtung nach außen) ausgelenkt werden kann.

Es erweist sich auch als vorteilhaft, wenn das Federelement eine Anlaufschräge für ein Löseinstrument aufweist. Hierbei kann es sich als vorteilhaft erweisen, wenn die Anlaufschräge von einer proximalen Seite des Abstützbereichs des Federelements gebildet ist.

Wenn das erste Federelement nach Art einer Wippe ausgebildet ist, so erweist es sich als vorteilhaft, wenn die Anlaufschräge von einer Außenseite des proximalen Wippenarms gebildet ist.

Die kanalbildende Zugangsöffnung kann in einer Wandung des betreffenden Schalenteils ausgebildet sein, sie kann aber auch nur abschnittsweise von dieser Wandung begrenzt sein. Sie kann in radialer Richtung nach außen abschnittsweise offen, insbesondere nutförmig, ausgebildet sein. Weiter kann die kanalbildende Zugangsöffnung entlang ihrer Erstreckung in der Längsrichtung abschnittsweise nach radial außen und abschnittsweise nach radial innen offen ausgebildet sein. Weiter kann die kanalbildende Zugangsöffnung mit einer radialen Durchgangsöffnung des Schalenteils kommunizieren.

Für alle vorstehend beschriebenen erfindungsgemäßen Ausführungsformen erweisen sich die folgenden Gestaltungsmerkmale als vorteilhaft:
Es erweist sich als vorteilhaft, dass das Schalenteil wenigstens einen und vorzugsweise zwei nach innen eingezogene Längsrandbereiche aufweist, mit dem es von in Umfangsrichtung gegenüberliegenden Seiten gegen einen Schenkel eines in der Seitenansicht U-förmigen Kopfs des Knochenankers anlegbar ist, so dass das Schalenteil hierdurch in der Umfangsrichtung unverdrehbar am Kopf des Knochenankers anordenbar ist.

Diese nach innen eingezogenen Längsrandbereiche können dann auch als Längsführungsmittel beim Fügen des Schalenteils an den Kopf des Knochenankers dienen.

Weiter erweist es sich als vorteilhaft, dass das Schalenteil eine erste Abstützkontaktfläche und eine zweite Abstützkontaktfläche aufweist und dass die erste Abstützkontaktfläche distal zu dem radialen Vorsprung zum Hintergreifen des hintergreifbaren Bereichs am Kopf des Knochenankers vorgesehen ist und dass die zweite Abstützkontaktfläche proximal zu dem radialen Vorsprung zum Hintergreifen des hintergreifbaren Bereichs am Kopf des Knochenankers vorgesehen ist, so dass das Schalenteil distal und proximal zu dem Vorsprung flächenhaft gegen eine Außenseite des Kopfs des Knochenankers anlegbar und abstützbar ist. Auf diese Weise lässt sich eine besonders vorteilhafte Stabilisierung des betreffenden Schalenteils gegen die Außenseite des Kopfs des Knochenankers erzielen. Da die Außenseite des Kopfs des Knochenankers und dementsprechend die erste und die zweite Abstützkontaktfläche des Schalenteils gekrümmt, insbesondere bogenförmig oder zylindrisch gekrümmt, ausgebildet sind, lässt sich ein Verkippen des Schalenteils relativ zum Kopf des Knochenankers vollständig vermeiden. Das Schalenteil ist dann bezüglich aller sechs Freiheitsgrade stabilisiert und unbewegbar am Kopf des Knochenankers gehalten, jedenfalls so lange wie die durch das Schwenkhebelelement ausgeübten Stellkräfte nicht bewusst und intendiert überwunden werden, um das Schalenteil wieder zu lösen.

Weiter erweist es sich als vorteilhaft, dass der radiale Vorsprung des jeweiligen Schalenteils in der Umfangsrichtung erstreckt ist und eine Neigung, Anschrägung oder Erstreckung in proximaler Richtung aufweist, so dass er mit einem ungefähr komplementär ausgebildeten und in der Umfangsrichtung erstreckten hintergreifbaren Bereich am Kopf des Knochenankers in der Längsrichtung und in der radialen Richtung einen Hintergriff ausbilden kann. Insbesondere erweist es sich als vorteilhaft, wenn der Vorsprung eine Umfangslänge aufweist, die der Umfangslänge des hintergreifbaren, typischerweise nutförmigen Bereichs am Kopf der Knochenschraube entspricht.

Gegenstand der Erfindung ist auch eine Sachgesamtheit aus einer Verlängerungsvorrichtung nach einem oder mehreren der vorstehenden Ansprüche und einem Knochenanker, insbesondere einer Pedikelschraube für die Wirbelsäulenchirurgie. Hierbei erweist sich als vorteilhaft, wenn der Knochenanker, insbesondere die Pedikelschraube, zwei in der Längsrichtung erstreckte Schenkel mit Innengewinde und je einer Sollbruchstelle aufweist, um die Schenkel nach der Implantation an der Sollbruchstelle abknicken und hierdurch kürzen zu können. Wenn ein solcher Knochenanker mit "Langkopf" verwendet wird, so lässt sich dessen verlängerter Innengewindebereich zum Einschrauben und Fixieren von weiteren Handhabungseinrichtungen und Instrumenten während der Implantation nutzen.

Weitere Merkmale, Einzelheiten und Vorteile der erfindungsgemäßen Verlängerungsvorrichtung ergeben sich aus den beigefügten Patentansprüchen und aus der zeichnerischen Darstellung und nachfolgenden Beschreibung erfindungsgemäßer Ausführungsformen der Erfindung.

In der Zeichnung zeigt:
Figur 1a eine perspektivische Ansicht eines Knochenankers in Form einer Pedikelschraube und einer erfindungsgemäßen Verlängerungsvorrichtung mit zwei Schalenteilen;
Figur 1b die Komponenten des Knochenankers der Figur 1a in einer lösbaren Halteverbindung;
Figuren 2a-f verschiedene Ansichten des Knochenankers nach Figur 1b;
Figuren 3a-f verschiedene Ansichten des Schalenteils des Knochenankers nach Figur 1a,b;
Figuren 4a-e eine Draufsicht und verschiedene Schnittansichten des Schalenteils des Knochenankers nach Figur 1a,b;
Figur 5 eine perspektivische Ansicht eines Schalenteils gemäß einer weiteren Ausführungsform der erfindungsgemäßen Verlängerungsvorrichtung;
Figuren 6a-c verschiedene Ansichten eines Schalenteils gemäß einer weiteren Ausführungsform der erfindungsgemäßen Verlängerungsvorrichtung;
Figuren 7a-e verschiedene Ansichten eines Schalenteils gemäß einer weiteren Ausführungsform der erfindungsgemäßen Verlängerungsvorrichtung;
Figuren 8a,b zwei perspektivische Ansichten eines Schalenteils gemäß einer weiteren Ausführungsform der erfindungsgemäßen Verlängerungsvorrichtung;

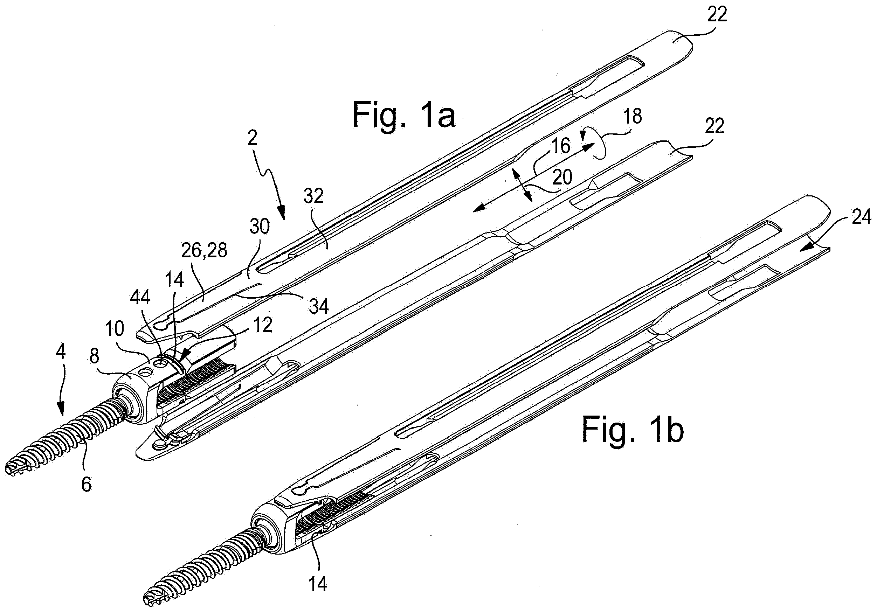

Die Figuren 1 bis 4 zeigen eine erste erfindungsgemäße Ausführungsform der erfindungsgemäßen Verlängerungsvorrichtung 2, teilweise in einer lösbaren Halteverbindung an einem Knochenanker 4 in Form einer beispielhaft dargestellten Pedikelschraube. Der Knochenanker 4 umfasst einen Schraubenschaft 6, der polyaxial verschwenkbar bezüglich eines bei einer seitlichen Betrachtung U-förmigen Kopfs 8 der Pedikelschraube 4 angeordnet ist. Bei dem Kopf 8 handelt es sich beispielhaft um einen sogenannten "Langkopf", der mit längeren Schenkeln 10 ausgebildet ist, wobei eine Sollbruchstelle 12 vorgesehen ist, an der der Langschaft im implantierten Zustand gekürzt werden kann. Dessen ungeachtet umfasst der Kopf 8 des Knochenankers 4 in an sich bekannter Weise einen hintergreifbaren Bereich 14, der in Form einer hintergreifbaren Nut am Außenumfang des Kopfs 8 ausgebildet ist. Der hintergreifbare Bereich 14 des Kopfs 8 kann im Übrigen entsprechend der Offenbarung in der Patentanmeldung DE 10 2014 225 327.6 der Anmelderin ausgebildet sein.

Die Verlängerungsvorrichtung 2 umfasst eine axiale Längsrichtung 16, eine hierzu konzentrische Umfangsrichtung 18 und eine radiale Richtung 20, die sich gleichermaßen auf den Kopf 8 des Knochenankers 4 beziehen. Die Verlängerungsvorrichtung 2 umfasst zwei voneinander separate und in der Längsrichtung 16 erstreckte Schalenteile 22, die beispielhaft und vorzugsweise einander entsprechend ausgebildet sind. Die Schalenteile 22 sind im Wesentlichen starr und verwindungsstabil ausgebildet in dem Sinne, dass sie nahezu vollständig formstabil sind, was aber eine geringfügige unbeachtliche Verformbarkeit insbesondere aufgrund der Länge nicht ausschließt. Die Schalenteile 22 sind auf noch zu beschreibende Weise lösbar an dem Kopf 8 des Knochenankers 4 befestigbar und bilden dann einen zwischen ihnen begrenzten Arbeitskanal 24, durch den der Chirurg mit weiteren Instrumenten Zugriff auf den Knochenanker 4 und das Operationsumfeld nehmen kann.

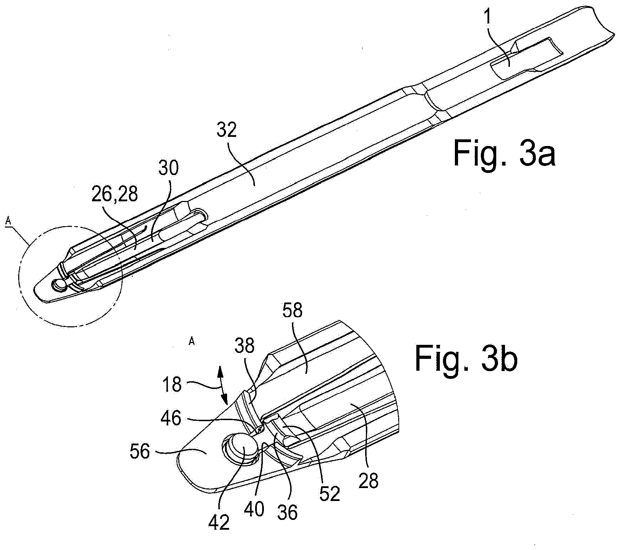

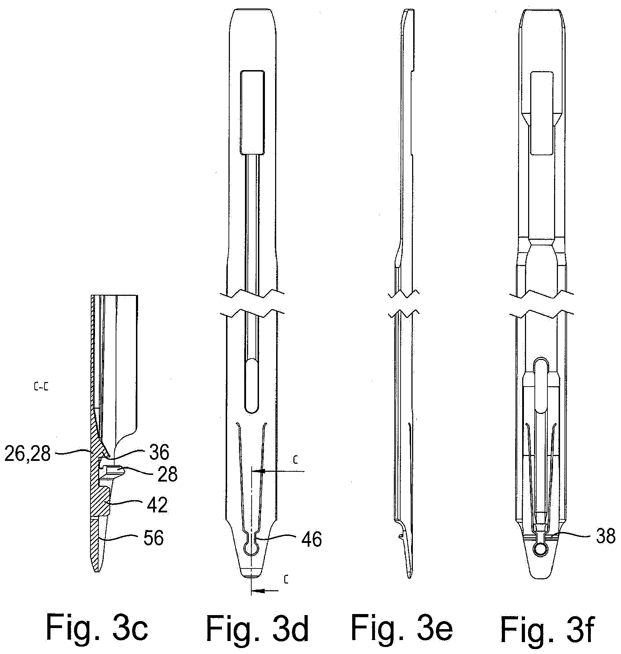

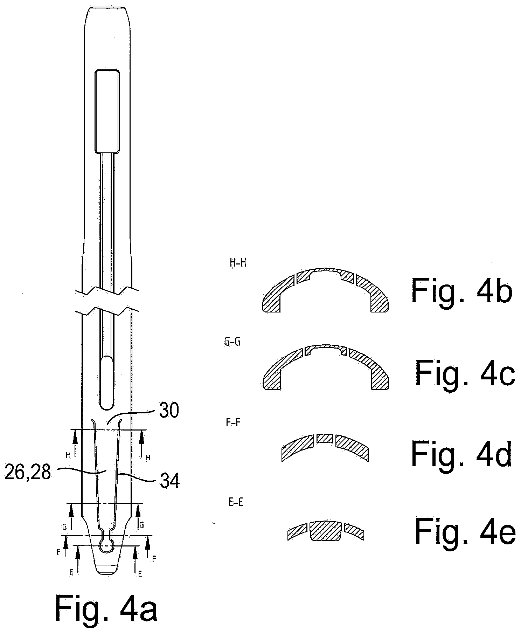

Ein jeweiliges Schaltenteil 22 ist im Wesentlichen formstabil ausgebildet, es umfasst jedoch einen ersten deformierbaren Bereich 26, der zugleich ein erstes Federelement 28 bildet, welches in der axialen Längsrichtung 16 erstreckt ist und quer hierzu ungefähr nach radial außen auslenkbar ist. Das Federelement 28 ist bei der dargestellten Ausführungsform beispielhaft einstückig mit dem Schalenteil 22 ausgebildet und geht an seinem proximalen Ende 30 einstückig in eine Wandung 32 des Schalenteils 22 über. Der erste deformierbare Bereich 26 beziehungsweise das Federelement 28 ist durch einen materialfreien Schlitz 34 in der Wandung 32 des Schalenteils 22 begrenzt. Der materialfreie Schlitz 34 läuft an seinen proximalen Enden gekrümmt aus, was eine gleichmäßige Spannungseinleitung in das Schalenteil 22 bewirkt. Man erkennt aus den Draufsicht der Figur 4a, dass das Federelement 28 in proximaler Richtung eine zunehmende Breite in Umfangsrichtung und eine zunehmende Dicke (Figuren 4b, 4c) aufweist. Das Federelement 28 weist weiter einen nach radial innen vorstehenden Abstützbereich 36 auf, mittels dessen das Federelement 28 und damit das Schalenteil 22 in axialer Längsrichtung 16 gegen den Kopf 8 des Knochenankers 4 abstützbar ist. Dieser Abstützbereich 36 ist in der Längsrichtung 16 proximal zu einem radialen Vorsprung 38 des Schalenteils 22 angeordnet. Der radiale Vorsprung 38 ist in der Umfangsrichtung 18 in Form einer nach innen vorstehenden Rippe ausgebildet, die auch eine Erstreckung in proximaler Richtung aufweist, um in den hintergreifbaren Bereich 14 am Kopf des Knochenankers eingreifen zu können. Man erkennt am besten in Figur 3b die Erstreckung des radialen Vorsprungs 38 in der Umfangsrichtung 18. Der radiale Vorsprung 38 bei der Ausführungsform nach Figuren 1 bis 4 weist eine mittige Unterbrechung 40 auf, damit das Federelement 28 sich in der Längsrichtung 16 distal durch den radialen Vorsprung 38 hindurch erstrecken kann. In dem Bereich distal bezüglich des radialen Vorsprungs 38 umfasst das Federelement 28 einen nach radial innen vorstehenden Eingriffsbereich 42, der in eine Eingriffsvertiefung 44 am Kopf 8 des Knochenankers 4 eingreifen kann und eine zusätzliche formschlüssige Verbindung zwischen dem Schalenteil 22 und dem Knochenanker 4 herstellen kann. Das Federelement 28 ist weiter so ausgebildet, dass es im Bereich der mittigen Unterbrechung 40 des radialen Vorsprungs 38 eine Verjüngung oder Einschnürung 46 ausweist, also dort in Umfangsrichtung schmäler ausgebildet ist.

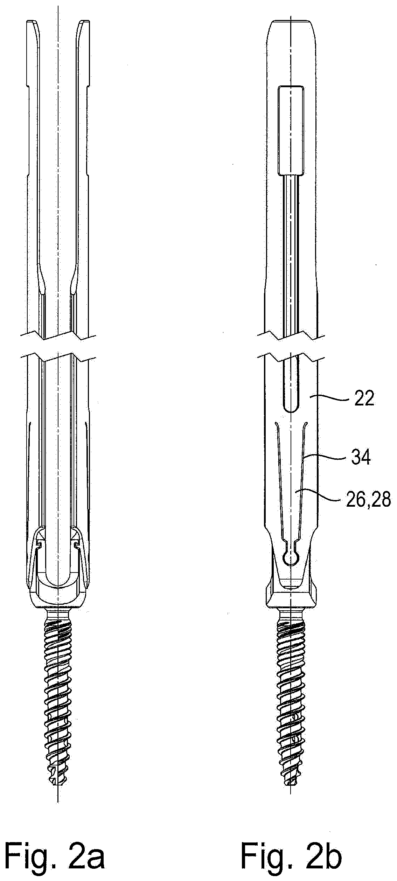

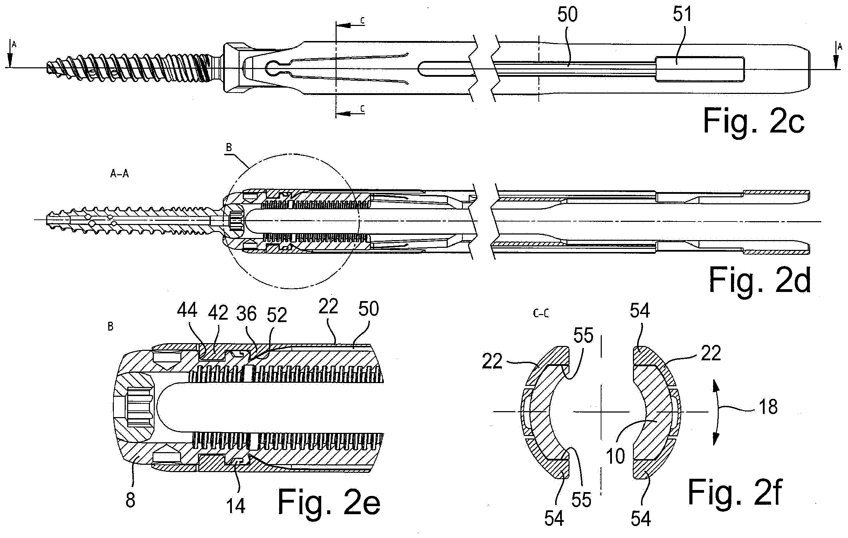

Wie eingangs beschrieben wird das betreffende Schalenteil 22 dadurch lösbar jedoch bezüglich aller Freiheitsgrade formschlüssig am Kopf 8 des Knochenankers 4 gehalten, das es, wie am besten aus Figuren 2e, f ersichtlich ist, mit seinem radialen Vorsprung 38 den hintergreifbaren Bereich 14 des Kopfs 8 des Knochenankers in der Längsrichtung 16 und in radialer Richtung 20 hinterhakt und sich in der entgegengesetzten Richtung mit seinem Abstützbereich 36 axial gegen den Kopf 8 abstützt. Bei der vorbeschriebenen Ausführungsform greift das Federelement 28 weiter mit seinem distalen Eingriffsbereich 42 in die Eingriffsvertiefung 44 am Kopf 8 ein. Aus Figur 2f ist ersichtlich, dass das jeweilige Schalenteil 22 zwei nach innen eingezogenen Längsrandbereiche 54 aufweist, mit denen es mit seitlichen Stützflächen 55 von in Umfangsrichtung 18 gegenüberliegenden Seiten gegen den Schenkel 10 des Kopfs 4 des Knochenankers anliegt. Das Schalenteil 22 umgreift also den Schenkel 10 im Wesentlichen wenigstens nahezu spielfrei und ist so auch in Umfangsrichtung 18 zusätzlich zu den eingreifenden Elementen unverdrehbar gehalten.

Insgesamt ist hier durch eine bezüglich aller Freiheitsgrade formschlüssige Betriebsverbindung geschaffen, die dadurch gelöst werden kann, das das Federelement 28 in radialer Richtung 20 nach außen ausgelenkt wird. Dies kann dadurch erreicht werden, dass mittels eines nicht dargestellten Löseinstruments Zugriff auf das Federelement 28 genommen wird. Hierfür ist bei dem jeweiligen Schalenteil 22 eine in der Längsrichtung 16 erstrecke kanalbildende Zugangsöffnung 50 ausgebildet, die entlang ihrer Längserstreckung in der Längsrichtung 16 abschnittsweise nach radial außen und abschnittsweise nach radial innen offen ausgebildet ist und auch durch das Federelement 28 selbst begrenzt ist. Die kanalbildende Zugangsöffnung durchläuft auch eine in Längsrichtung 16 ausladende radiale Durchgangsöffnung 51 in dem Schalenteil 22. Im beispielhaften dargestellten Fall weist das Federelement eine Anlaufschräge 52 für das Löseinstrument auf, und zwar an seiner Innenseite und an einer proximalen Seite seines Abstützbereichs 36.

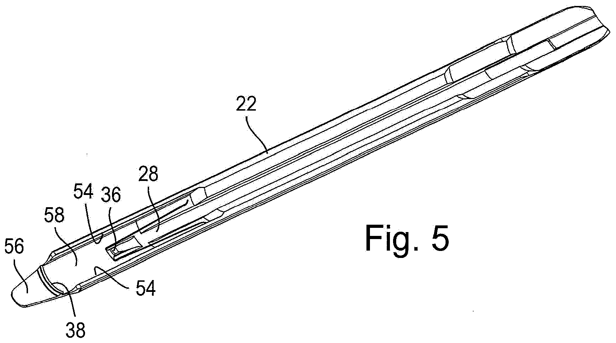

Figur 5 zeigt eine gegenüber der vorbeschriebenen Ausführungsform reduzierte Ausführungsform, bei der das Federelement 28 in distaler Richtung nicht über den radialen Vorsprung 38 erstreckt ist. Ferner ist vorzugsweise und beispielhaft ersichtlich, dass ein jeweiliges Schalenteil 22 eine erste Abstützkontaktfläche 56 und eine zweite Abstützkontaktfläche 58 aufweist, die distal beziehungsweise proximal zu dem radialen Vorsprung 36 ausgebildet sind. Sie sind entsprechend der Wölbung der Außenseite des Kopfs 8 ausgebildet, so dass das Schalenteil 22 distal und proximal zu dem Vorsprung 36 flächenhaft gegen die Außenseite des Kopfs des Knochenankers anlegbar und abstützbar ist. Dies bewirkt zusätzlich im Zusammenwirken mit den formschlüssigen Hintergriffen eine zusätzliche Stabilisierung der Halteverbindung.

Das Schalenteil 22 nach Figur 5 ist also durch den Eingriff seines radialen Vorsprungs 38 in den hintergreifbaren Bereich 14 am Kopf des Knochenankers sowie durch die axiale Abstützung durch den Abstützbereich 36 gegen den Kopf des Knochenankers fixierbar. Dabei bilden die seitlichen Längsrandbereiche 54 einen Formschluss mit dem jeweiligen Schenkel 10 des Kopfs des Knochenankers, und die Abstützkontaktflächen 56, 58 stabilisieren die Halteverbindung zusätzlich.

Das weitere Ausführungsbeispiel gemäß Figuren 6a bis c geht aus von der Ausführungsform nach Figur 5. Das hier dargestellte Schalenteil 22 weist zusätzlich einen zweiten deformierbaren Bereich 60 auf, der ein zweites Federelement 62 mit einem nach radial innen vorstehenden Eingriffsbereich 64 bildet. Der zweite deformierbare Bereich 60 ist vorliegend distal zu dem radialen Vorsprung 38 ausgebildet und von einem sich verjüngenden distalen Endabschnitt 66 des Schalenteils 22 gebildet, der quer zur Längsrichtung federnd ausgebildet ist. Dies kann durch Schwächungen in Form von Nuten oder Schlitzen (nicht dargestellt) oder eine geringere Wandstärke als in anschließenden im Wesentlichen starren Bereichen des Schalenteils 22 realisiert sein.

Bei der beispielhaften Ausführungsform nach Figuren 7a bis e ist ebenfalls zusätzlich zu dem ersten deformierbaren Bereich 26 mit erstem Federelement 28 ein zweiter deformierbarer Bereich 60 mit zweitem Federelement 62 vorgesehen. Das zweite Federelement 62 ist hier U-förmig ausgebildet und umfasst zwei Schenkel 68 die in der Längsrichtung 16 erstreckt sind, und einen diese verbindenden Quersteg 70. An diesem Quersteg 70 ist wiederum ein Eingriffsbereich 64 ausgebildet. Das Federelement 62 ist mit seinen Schenkeln 68 einstückig an das Schalenteil 22 angebunden.

Bei der Ausführungsform nach Figuren 8a, b ist der erste deformierbare Bereich 26 beziehungsweise das erste Federelement 28 nach Art einer Wippe ausgebildet und weist einen bezüglich einer Schwenkachse 72 distalen Wippenarm 74 und einen diesbezüglich proximalen Wippenarm 76 auf. Am proximalen Wippenarm 76 ist der nach radial innen vorstehende Abstützbereich 36 ausgebildet. Auch bei dieser Ausführungsform ist beispielhaft ein zweiter deformierbarer Bereich 60 vorgesehen, der ein zweites Federelement 62 mit einem Eingriffsbereich 64 bildet. Ferner sind der Wölbung der Außenseite des Kopfs des Knochenankers entsprechende Abstützkontaktflächen 56, 58 vorgesehen.

## Patentansprüche

1. Verlängerungsvorrichtung (2) für einen Knochenanker (4), insbesondere für eine Knochenschraube, insbesondere für eine Pedikelschraube, insbesondere in der minimalinvasiven Wirbelsäulenchirurgie, mit einer axialen Längsrichtung (16), einer hierzu konzentrischen Umfangsrichtung (18) und einer radialen Richtung (20), wobei die Verlängerungsvorrichtung (2) in der axialen Längsrichtung (16) erstreckt ist und mittels eines radialen Vorsprungs (38) einen hintergreifbaren Bereich (14) am Kopf (8) des Knochenankers (4) in der Längsrichtung (16) und zudem in der radialen Richtung (20) hintergreift und in entgegengesetzter Richtung gegen den Kopf (8) des Knochenankers (4) abstützbar ist, so dass die Verlängerungsvorrichtung hierdurch am Kopf (8) des Knochenankers lösbar, jedoch in der Längsrichtung (16) starr und zudem drehfest fixierbar ist, **dadurch gekennzeichnet, dass** die Verlängerungsvorrichtung (2) wenigstens ein und vorzugsweise zwei voneinander separate, und in der Längsrichtung (16) erstreckte Schalenteile (22) umfasst, von denen jedes einen radialen Vorsprung (38) zum Hintergreifen des hintergreifbaren Bereichs (14) am Kopf (8) des Knochenankers (4) umfasst,
dass das Schalenteil (22) überwiegend im Wesentlichen starr ausgebildet ist, jedoch einen ersten deformierbaren Bereich (26) aufweist,
dass der erste deformierbare Bereich (26) ein in der axialen Längsrichtung (16) erstrecktes und quer hierzu, insbesondere im Wesentlichen radial nach außen, auslenkbares Federelement (28) bildet,
dass das Federelement (28) proximal zu dem radialen Vorsprung (38) zum Hintergreifen des hintergreifbaren Bereichs (14) am Kopf (8) des Knochenankers einen nach radial innen vorstehenden Abstützbereich (36) aufweist, mittels dessen das Federelement (28) und damit das Schalenteil (22) in axialer Längsrichtung (16) gegen den Kopf (8) des Knochenankers abstützbar ist, so dass die Verlängerungsvorrichtung hierdurch am Kopf (8) des Knochenankers lösbar fixierbar ist, wenn der Vorsprung (38) den hintergreifbaren Bereich (14) am Kopf (8) des Knochenankers (4) hintergreift, da durch die axiale Abstützung mittels des Abstützbereichs (36) verhindert ist, dass sich der radiale Vorsprung (38) des Schalenteils (22) aus dem hintergreifbaren Bereich (14) am Kopf (8) des Knochenankers (4) in distaler Richtung löst, unddass das Federelement (28) zum Lösen der Verlängerungsvorrichtung (2) radial nach außen auslenkbar ist, so dass sein Abstützbereich (36) vom Kopf (8) des Knochenankers frei kommt und das Schalenteil (22) gegenüber dem Kopf (8) des Knochenankers aus dem hintergreifbaren Bereich (14) am Kopf (8) des Knochenankers in der Längsrichtung (16) distal herausbewegt werden kann.

2. Verlängerungsvorrichtung nach Anspruch 1, **dadurch gekennzeichnet, dass** das Federelement (28) einstückig mit dem Schalenteil (22) ausgebildet ist und an seinem proximalen Ende (30) in eine Wandung (32) des Schalenteils (22) übergeht, und insbesondere das Federelement (28) durch einen materialfreien Schlitz (34) in der Wandung (32) des Schadenteils (22) begrenzt ist und an seinem proximalen Ende (30) einstückig in die Wandung (32) des Schalenteils (22) übergeht.

3. Verlängerungsvorrichtung nach Anspruch 2, **dadurch gekennzeichnet, dass** der materialfreie Schlitz (34) an seinem Ende (30) in eine gegenüber der Schlitzbreite größere Öffnung ausmündet.

4. Verlängerungsvorrichtung nach einem oder mehreren der vorstehenden Ansprüche, **dadurch gekennzeichnet, dass** das Federelement (28) in einem Bereich seines Federarms proximal zu dem nach radial innen vorstehenden Abstützbereich (36) eine in der axialen Längsrichtung (16) in proximaler Richtung zunehmende Wanddicke aufweist.

5. Verlängerungsvorrichtung nach einem oder mehreren der vorstehenden Ansprüche, **dadurch gekennzeichnet, dass** das Federelement (28) in der Längsrichtung (16) distal bis über den radialen Vorsprung (38) zum Hintergreifen des hintergreifbaren Bereichs (14) am Kopf (8) des Knochenankers erstreckt ist und dort einen nach radial innen vorstehenden Eingriffsbereich (42) aufweist, der in eine Eingriffsvertiefung (44) am Kopf (8) des Knochenankers einrastbar ist und eine zusätzliche formschlüssige Verbindung zwischen dem Schalenteil (22) und dem Kopf (8) des Knochenankers herstellen kann.

6. Verlängerungsvorrichtung nach Anspruch 5, **dadurch gekennzeichnet, dass** der radiale Vorsprung (38) zum Hintergreifen des hintergreifbaren Bereichs (14) am Kopf (8) des Knochenankers in der Umfangsrichtung (18) erstreckt ist, und dass dieser radiale Vorsprung (38) und eine Wandung (32) des Schalenteils (22) im Bereich des Federelements (28) in Umfangsrichtung (18) unterbrochen oder ausgespart ist, damit das Federelement (28) sich in der axialen Längsrichtung dort hindurch erstrecken kann.

7. Verlängerungsvorrichtung nach Anspruch 5 oder 6,
**dadurch gekennzeichnet, dass** das Federelement (28) im Bereich des radialen Vorsprungs (38) zum Hintergreifen des hintergreifbaren Bereichs (14) am Kopf (8) des Knochenankers in Umfangsrichtung (18) verjüngt oder eingeschnürt ausgebildet ist.

8. Verlängerungsvorrichtung nach einem oder mehreren der vorstehenden Ansprüche, **dadurch gekennzeichnet, dass** das Schalenteil (22) distal zu dem radialen Vorsprung (38) zum Hintergreifen des hintergreifbaren Bereichs (14) am Kopf (8) des Knochenankers einen zweiten deformierbaren Bereich (60) aufweist, der ein zweites Federelement (62) bildet, wobei das zweite Federelement (62) einen nach radial innen vorstehenden Eingriffsbereich (64) aufweist, der in eine Eingriffsvertiefung (44) am Kopf (8) des Knochenankers (4) einrastbar ist und eine zusätzliche formschlüssige Verbindung zwischen dem Schalenteil (22) und dem Kopf (8) des Knochenankers herstellen kann.

9. Verlängerungsvorrichtung nach Anspruch 8, **dadurch gekennzeichnet, dass** der zweite deformierbare Bereich (60) des Schalenteils (22) von einem sich verjüngenden distalen Endabschnitt (66) des Schalenteils (22) gebildet ist.

10. Verlängerungsvorrichtung nach einem oder mehreren der vorstehenden Ansprüche, **dadurch gekennzeichnet, dass** der erste deformierbare Bereich (26) und das von ihm gebildete Federelement (28) nach Art einer Wippe ausgebildet ist und einen bezüglich einer Schwenkachse (72) distalen Wippenarm (74) und einen bezüglich der Schwenkachse (72) proximalen Wippenarm (76) aufweist und dass das Federelement in eine Freigabestellung bringbar ist, indem der proximale Wippenarm (76) nach radial innen ausgelenkt wird, so dass hierdurch der distale Wippenarm (74) nach radial außen ausgelenkt wird und somit vom Kopf (8) des Knochenankers (4) frei kommt.

11. Verlängerungsvorrichtung nach einem oder mehreren der vorstehenden Ansprüche, **dadurch gekennzeichnet, dass** das Schalenteil (22) eine in der Längsrichtung (16) erstreckte kanalbildende Zugangsöffnung (50) aufweist, durch welche mittels eines Löseinstruments Zugang zu dem Federelement genommen werden kann und das Federelement (28) zum Lösen der Verlängerungsvorrichtung ausgelenkt werden kann und dass das Federelement (28) eine Anlaufschräge (52) für ein Löseinstrument aufweist, die insbesondere von einer proximalen Seite des Abstützbereichs (36) des Federelements (28) gebildet ist.

12. Verlängerungsvorrichtung nach einem oder mehreren der vorstehenden Ansprüche, **dadurch gekennzeichnet, dass** das Schalenteil wenigstens einen und vorzugsweise zwei nach innen eingezogene Längsrandbereiche (54) aufweist, mit dem es von in Umfangsrichtung (18) gegenüberliegenden Seiten gegen einen Schenkel (10) eines in der Seitenansicht U-förmigen Kopfs (8) des Knochenankers (4) anlegbar ist, so dass das Schalenteil (22) hierdurch in der Umfangsrichtung (18)unverdrehbar am Kopf (8) des Knochenankers anordenbar ist.

13. Verlängerungsvorrichtung nach einem oder mehreren der vorstehenden Ansprüche, **dadurch gekennzeichnet, dass** das Schalenteil (22) eine erste Abstützkontaktfläche (56) und eine zweite Abstützkontaktfläche (58) aufweist und dass die erste Abstützkontaktfläche (56) distal zu dem radialen Vorsprung (38) zum Hintergreifen des hintergreifbaren Bereichs (14) am Kopf des Knochenankers vorgesehen ist und dass die zweite Abstützkontaktfläche (58) proximal zu dem radialen Vorsprung (38) zum Hintergreifen des hintergreifbaren Bereichs (14) am Kopf des Knochenankers vorgesehen ist, so dass das Schalenteil (22) distal und proximal zu dem Vorsprung (38) flächenhaft gegen eine Außenseite des Kopfs (8) des Knochenankers anlegbar und abstützbar ist.

14. Verlängerungsvorrichtung nach einem oder mehreren der vorstehenden Ansprüche, **dadurch gekennzeichnet, dass** der radiale Vorsprung (38) des jeweiligen Schalenteils (22) in der Umfangsrichtung (18) erstreckt ist und eine Neigung, Anschrägung oder Erstreckung in proximaler Richtung aufweist, so dass er mit einem ungefähr komplementär ausgebildeten und in der Umfangsrichtung erstreckten hintergreifbaren Bereich (14) am Kopf (8) des Knochenankers (4) in der Längsrichtung (16) und in der radialen Richtung (20) einen Hintergriff ausbilden kann.

15. Sachgesamtheit aus einer Verlängerungsvorrichtung (2) nach einem oder mehreren der vorstehenden Ansprüche und einem Knochenanker (4), insbesondere eine Pedikelschraube für die Wirbelsäulenchirurgie.

## Claims

1. Extension device (2) for a bone anchor (4), in particular for a bone screw, in particular for a pedicle screw, in particular in minimally invasive spinal surgery, having an axial longitudinal direction (16), a peripheral direction (18) concentric therewith, and a radial direction (20), the extension device (2) extending in the axial longitudinal direction (16) and engaging, by means of a radial projection (38), an engageable region (14) on the head (8) of the bone anchor (4) in the longitudinal direction (16) and also in the radial direction (20) and being supportable against the head (8) of the bone anchor (4) in the opposite direction such that the extension device can thereby be fixed to the head (8) of the bone anchor so as to be detachable, yet also rigid in the longitudinal direction (16) and non-rotatable, **characterized in that** the extension device (2) comprises at least one, preferably two separate shell parts (22) that extend in the longitudinal direction (16), each of which parts comprises a radial projection (38) for engaging the engageable region (14) on the head (8) of the bone anchor (4), **in that** the shell part (22) is, for the most part, substantially rigid, but has a first deformable region (26), **in that** the first deformable region (26) forms a spring element (28) which extends in the axial longitudinal direction (16) and can be deflected transversely to said direction, in particular substantially radially outwardly, **in that** the spring element (28) comprises, proximally to the radial projection (38) for engaging the engageable region (14) on the head (8) of the bone anchor, a radially inwardly projecting support region (36), by means of which the spring element (28) and thus the shell part (22) is supportable against the head (8) of the bone anchor in the axial longitudinal direction (16) such that the extension device can thereby be detachably fixed to the head (8) of the bone anchor when the projection (38) engages the engageable region (14) on the head (8) of the bone anchor (4), as the axial support by means of the support region (36) prevents the radial projection (38) of the shell part (22) from disengaging in the distal direction from the engageable area (14) on the head (8) of the bone anchor (4) in the distal direction and **in that** the spring element (28) is deflectable radially outwardly so as to detach the extension device (2), such that the support region (36) of said spring element is freed from the head (8) of the bone anchor and the shell part (22) can be moved relative to the head (8) of the bone anchor distally out of the engageable region (14) on the head (8) of the bone anchor in the longitudinal direction (16).

2. Extension device according to claim 1, **characterized in that** the spring element (28) is integral with the shell part (22) and transitions, at its proximal end (30), into a wall (32) of the shell part (22) and in particular the spring element (28) is delimited by a material-free slot (34) in the wall (32) of the shell part (22) and integrally transitions, at its proximal end (30), into the wall (32) of the shell part (22).

3. Extension device according to claim 2, **characterized in that** the material-free slot (34) leads, at its end (30), into an opening that is larger than the slot width.

4. Extension device according to one or more of the preceding claims, **characterized in that** the spring element (28) has, in a region of its spring arm proximal to the radially inwardly projecting support region (36), a wall thickness that increases in the axial longitudinal direction (16) in the proximal direction.

5. Extension device according to one or more of the preceding claims, **characterized in that** the spring element (28) extends in the longitudinal direction (16) distally beyond the radial projection (38) for engaging the engageable region (14) on the head (8) of the bone anchor, and comprises at this point a radially inwardly projecting engagement portion (42) which can be latched into an engagement recess (44) in the head (8) of the bone anchor and can establish an additional interlocking connection between the shell part (22) and the head (8) of the bone anchor.

6. Extension device according to claim 5, **characterized in that** the radial projection (38) for engaging the engageable region (14) on the head (8) of the bone anchor extends in the peripheral direction (18), and **in that** this radial projection (38) and a wall (32) of the shell part (22) has gaps or recesses in the peripheral direction (18) in the region of the spring element (28) so that the spring element (28) can then extend therethrough in the axial longitudinal direction.

7. Extension device according to claim 5 or 6, **characterized in that** the spring element (28) is tapered or necked in the peripheral direction (18) in the region of the radial projection (38) for engaging the engageable region (14) on the head (8) of the bone anchor.

8. Extension device according to one or more of the preceding claims, **characterized in that** the shell part (22) comprises, distally to the radial projection (38) for engaging the engageable region (14) on the head (8) of the bone anchor, a second deformable region (60), which forms a second spring element (62), the second spring element (62) comprising a radially inwardly projecting engagement region (64) which can be latched into an engagement recess (44) in the head (8) of the bone anchor (4) and can establish an additional interlocking connection between the shell part (22) and the head (8) of the bone anchor.

9. Extension device according to claim 8, **characterized in that** the second deformable region (60) of the shell part (22) is formed by a tapered distal end portion (66) of the shell part (22).

10. Extension device according to one or more of the preceding claims, **characterized in that** the first deformable region (26) and the first spring element (28) formed thereby is designed in the manner of a rocker and has a rocker arm (74) which is distal to a pivot pin (72) and a rocker arm (76) which is proximal to the pivot pin (72) and that the spring element can be brought into a release position by the proximal rocker arm (76) being deflected radially inwardly so that the distal rocker arm (74) is thereby deflected radially outwardly and is thus freed from the head (8) of the bone anchor (4).

11. Extension device according to one or more of the preceding claims, **characterized in that** the shell part (22) has a channel-forming access opening (50) which extends in the longitudinal direction (16), through which opening access to the spring element can be gained by means of a release instrument and the spring element (28) can be deflected so as to detach the extension device and that the spring element (28) has an inclined run-on surface (52) for a release instrument, wherein in particular the inclined run-on surface (52) is formed by a proximal side of the support region (36) of the spring element (28).

12. Extension device according to one or more of the preceding claims, **characterized in that** the shell part has at least one, preferably two inwardly directed longitudinal edge regions (54), by means of which said part can be positioned against a leg (10) of a head (8) of the bone anchor (4) that is U-shaped in a side view by sides that are opposite in the peripheral direction (18), such that the shell part (22) can be thereby arranged on the head (8) of the bone anchor such that it cannot rotate in the peripheral direction (18).

13. Extension device according to one or more of the preceding claims, **characterized in that** the shell part (22) has a first support contact surface (56) and a second support contact surface (58), and **in that** the first support contact surface (56) is provided distally to the radial projection (38) for engaging the engageable region (14) on the head of the bone anchor and the second support contact surface (58) is provided proximally to the radial projection (38) for engaging the engageable region (14) on the head of the bone anchor such that the shell part (22) can be extensively positioned and supported against an outer side of the head (8) of the bone anchor distally and proximally to the projection (38) .

14. Extension device according to one or more of the preceding claims, **characterized in that** the radial projection (38) of the relevant shell part (22) extends in the peripheral direction (18) and has an inclination, bevel or extension in the proximal direction such that it can form an engagement in the longitudinal direction (16) and radial direction (20) with an engageable region (14) on the head (8) of the bone anchor (4), which region is approximately complementary to said projection and extends in the peripheral direction.

15. Assembly consisting of an extension device (2) according to one or more of the preceding claims and a bone anchor (4), in particular a pedicle screw for spinal surgery.

## Revendications

1. Dispositif de prolongement (2) pour une ancre à os (4), en particulier pour une vis à os, en particulier pour une vis pédiculaire, en particulier en chirurgie mini-invasive de la colonne vertébrale, ayant une direction longitudinale axiale (16), une direction circonférentielle (18) concentrique à celle-ci et une direction radiale (20), dans lequel le dispositif de prolongement (2) s'étend dans la direction longitudinale axiale (16) et s'engage au moyen d'une saillie radiale (38), dans la direction longitudinale (16) et en sus dans la direction radiale (20), derrière une zone (14) sur la tête (8) de l'ancre à os (4), laquelle est apte à l'engagement de derrière, et peut être appuyé dans la direction opposée contre la tête (8) de l'ancre à os (4), de sorte que, ainsi, le dispositif de prolongement peut être fixé de manière amovible sur la tête (8) de l'ancre à os, mais de manière rigide dans la direction longitudinale (16) et en sus fixé de manière solidaire en rotation, **caractérisé par le fait que** le dispositif de prolongement (2) comprend au moins une partie de coque et de préférence deux parties de coque (22) séparées l'une de l'autre et s'étendant dans la direction longitudinale (16), dont chacune présente une saillie radiale (38) destinée à s'engager derrière la zone (14) sur la tête (8) de l'ancre à os (4), laquelle est apte à l'engagement de derrière,
que la partie de coque (22) est conçue principalement pour l'essentiel de manière rigide, mais présente une première zone déformable (26),
que la première zone déformable (26) forme un élément faisant ressort (28) qui s'étend dans la direction longitudinale axiale (16) et peut être dévié transversalement à celle-ci, en particulier pour l'essentiel radialement vers l'extérieur,
que ledit élément faisant ressort (28) présente de manière proximale par rapport à la saillie radiale (38) destinée à s'engager derrière la zone (14) sur la tête (8) de l'ancre à os, laquelle est apte à l'engagement de derrière, une zone d'appui (36) faisant saillie radialement vers l'intérieur et au moyen de laquelle l'élément faisant ressort (28) et donc la partie de coque (22) peuvent être appuyés dans la direction longitudinale axiale (16) contre la tête (8) de l'ancre à os, de sorte que, ainsi, le dispositif de prolongement peut être fixé de manière amovible sur la tête (8) de l'ancre à os lorsque la saillie (38) s'engage derrière la zone (14) sur la tête (8) de l'ancre à os (4), laquelle est apte à l'engagement de derrière, puisque l'appui axial au moyen de la zone d'appui (36) empêche la saillie radiale (38) de la partie de coque (22) de sortir dans la direction distale de la zone (14) sur la tête (8) de l'ancre à os (4), laquelle est apte à l'engagement de derrière, et que l'élément faisant ressort (28) peut être dévié radialement vers l'extérieur pour libérer le dispositif de prolongement (2), de sorte que sa zone d'appui (36) est dégagée de la tête (8) de l'ancre à os et que la partie de coque (22) peut être déplacée, par rapport à la tête (8) de l'ancre à os, distalement dans la direction longitudinale (16) hors de la zone (14) sur la tête (8) de l'ancre à os, laquelle est apte à l'engagement de derrière.

2. Dispositif de prolongement selon la revendication 1, **caractérisé par le fait que** l'élément faisant ressort (28) est réalisé d'une seule pièce avec la partie de coque (22) et se confond, à son extrémité proximale (30), avec une paroi (32) de la partie de coque (22), et que, en particulier, l'élément faisant ressort (28) est délimité par une fente exempte de matière (34) dans la paroi (32) de la partie de coque (22) et se confond, à son extrémité proximale (30), en une seule pièce avec la paroi (32) de la partie de coque (22).

3. Dispositif de prolongement selon la revendication 2, **caractérisé par le fait que** la fente exempte de matière (34) débouche, à son extrémité (30), dans une ouverture plus grande que la largeur de la fente.

4. Dispositif de prolongement selon l'une ou plusieurs des revendications précédentes, **caractérisé par le fait que**, au niveau d'une zone de son bras faisant ressort, de manière proximale par rapport à la zone d'appui (36) faisant saillie radialement vers l'intérieur, l'élément faisant ressort (28) présente une épaisseur de paroi augmentant dans la direction longitudinale axiale (16) dans la direction proximale.

5. Dispositif de prolongement selon l'une ou plusieurs des revendications précédentes, **caractérisé par le fait que** l'élément faisant ressort (28) s'étend dans la direction longitudinale (16) distalement jusqu'au-delà de la saillie radiale (38) destinée à s'engager derrière la zone (14) sur la tête (8) de l'ancre à os, laquelle est apte à l'engagement de derrière, et présente là une zone d'engagement (42) faisant saillie radialement vers l'intérieur qui peut être encliquetée dans un évidement d'engagement (44) sur la tête (8) de l'ancre à os et peut réaliser une liaison à engagement positif supplémentaire entre la partie de coque (22) et la tête (8) de l'ancre à os.

6. Dispositif de prolongement selon la revendication 5, **caractérisé par le fait que** la saillie radiale (38) destinée à s'engager derrière la zone (14) sur la tête (8) de l'ancre à os, laquelle est apte à l'engagement de derrière, s'étend dans la direction circonférentielle (18) et que cette saillie radiale (38) et une paroi (32) de la partie de coque (22) sont interrompues ou évidée au niveau de l'élément faisant ressort (28) dans la direction circonférentielle (18) pour que l'élément faisant ressort (28) puisse s'y étendre à travers dans la direction longitudinale axiale.

7. Dispositif de prolongement selon la revendication 5 ou 6, **caractérisé par le fait que** l'élément faisant ressort (28) est réalisé de manière rétrécie ou resserrée dans la direction circonférentielle (18) au niveau de la saillie radiale (38) destinée à s'engager derrière la zone (14) sur la tête (8) de l'ancre à os, laquelle est apte à l'engagement de derrière.

8. Dispositif de prolongement selon l'une ou plusieurs des revendications précédentes, **caractérisé par le fait que** la partie de coque (22) présente de manière distale par rapport à la saillie radiale (38) destinée à s'engager derrière la zone (14) sur la tête (8) de l'ancre à os, laquelle est apte à l'engagement de derrière, une deuxième zone déformable (60) qui forme un deuxième élément faisant ressort (62), dans lequel le deuxième élément faisant ressort (62) présente une zone d'engagement (64) faisant saillie radialement vers l'intérieur qui peut être encliquetée dans un évidement d'engagement (44) sur la tête (8) de l'ancre à os (4) et peut réaliser une liaison à engagement positif supplémentaire entre la partie de coque (22) et la tête (8) de l'ancre à os.

9. Dispositif de prolongement selon la revendication 8, **caractérisé par le fait que** la deuxième zone déformable (60) de la partie de coque (22) est formée par un tronçon d'extrémité distal (66) de la partie de coque (22), qui se rétrécit.

10. Dispositif de prolongement selon l'une ou plusieurs des revendications précédentes, **caractérisé par le fait que** la première zone déformable (26) et l'élément faisant ressort (28) formé par celle-ci sont conçus à la manière d'une bascule et présentent un bras de bascule (74) distal par rapport à un axe de pivotement (72) et un bras de bascule (76) proximal par rapport à l'axe de pivotement (72) et que l'élément faisant ressort peut être amené dans une position de libération en déviant le bras proximal de bascule (76) radialement vers l'intérieur de sorte que, ainsi, le bras distal de bascule (74) est dévié radialement vers l'extérieur et est ainsi libéré de la tête (8) de l'ancre à os (4).

11. Dispositif de prolongement selon l'une ou plusieurs des revendications précédentes, **caractérisé par le fait que** la partie de coque (22) présente une ouverture d'accès (50) formant un canal et s'étendant dans la direction longitudinale (16), à travers laquelle on peut avoir accès, au moyen d'un instrument de libération, à l'élément faisant ressort et l'élément faisant ressort (28) peut être dévié pour libérer le dispositif de prolongement et que l'élément faisant ressort (28) présente une pente d'entrée (52) pour un instrument de libération, qui est formée en particulier par un côté proximal de la zone d'appui (36) de l'élément faisant ressort (28).

12. Dispositif de prolongement selon l'une ou plusieurs des revendications précédentes, **caractérisé par le fait que** la partie de coque présente au moins une et de préférence deux zones marginales longitudinales rentrantes (54) par laquelle/lesquelles elle peut être appliquée, depuis des côtés opposés dans la direction circonférentielle (18), contre une branche (10) d'une tête (8) de l'ancre à os (4), qui est en forme d'U dans la vue latérale, de sorte que, ainsi, la partie de coque (22) peut être disposée sur la tête (8) de l'ancre à os de manière à ne pas pouvoir être tournée dans la direction circonférentielle (18).

13. Dispositif de prolongement selon l'une ou plusieurs des revendications précédentes, **caractérisé par le fait que** la partie de coque (22) comprend une première surface de contact d'appui (56) et une deuxième surface de contact d'appui (58) et que la première surface de contact d'appui (56) est prévue de manière distale par rapport à la saillie radiale (38) destinée à s'engager derrière la zone (14) sur la tête de l'ancre à os, laquelle est apte à l'engagement de derrière, et que la deuxième surface de contact d'appui (58) est prévue de manière proximale par rapport à la saillie radiale (38) destinée à s'engager derrière la zone (14) sur la tête de l'ancre à os, laquelle est apte à l'engagement de derrière, de sorte que la partie de coque (22) peut être appliquée et appuyée, de manière distale et proximale par rapport à la saillie (38), en nappe contre une face extérieure de la tête (8) de l'ancre à os.

14. Dispositif de prolongement selon l'une ou plusieurs des revendications précédentes, **caractérisé par le fait que** la saillie radiale (38) de la partie de coque (22) respective s'étend dans la direction circonférentielle (18) et présente une inclinaison, un biseau ou une extension dans la direction proximale de sorte qu'elle peut réaliser un engagement de derrière dans la direction longitudinale (16) et dans la direction radiale (20) avec une zone (14) sur la tête (8) de l'ancre à os (4), qui est réalisée de façon à peu prés complémentaire, s'étend dans la direction circonférentielle et est apte à l'engagement de derrière.

15. Ensemble matériel composé d'un dispositif de prolongement (2) selon l'une ou plusieurs des revendications précédentes et d'un ancre à os (4), en particulier d'une vis pédiculaire pour la chirurgie de la colonne vertébrale.
